# EUROPÄISCHE PATENTSCHRIFT

(11) **EP 3 151 888 B1**
(45) Veröffentlichungstag und Bekanntmachung des Hinweises auf die Patenterteilung: **16.05.2018**
(21) Anmeldenummer: 15731842.9
(22) Anmeldetag: 27.04.2015
(51) Int. Cl.: A61M 5/315, A61M 5/32, A61M 5/46, A61M 5/20, A61M 5/31, A61M 5/145

(54) **INJEKTIONSVORRICHTUNG MIT ZAHNRADGETRIEBE**
INJECTION DEVICE WITH TOOTHED GEARING
DISPOSITIF D'INJECTION MUNI D'UN ROUAGE

(30) Priorität: 03.06.2014 DE 202014004561 U
(43) Veröffentlichungstag der Anmeldung: 12.04.2017
(73) Patentinhaber: H & B Electronic GmbH & Co. KG, 75392 Deckenpfronn (DE)
(72) Erfinder: MORLOK, Tobias, 71159 Mötzingen (DE); WEBER, Wilfried, 72296 Schopfloch (DE)
(74) Vertreter: Wacker, Jost Oliver
(86) Internationale Anmeldenummer: PCT/EP2015/000863
(87) Internationale Veröffentlichungsnummer: WO 2015/185176

(56) Entgegenhaltungen:
- FR-A1- 2 767 479
- US-A1- 2011 028 910

## Beschreibung

Die Erfindung betrifft eine Injektionsvorrichtung für eine vereinfachte Verabreichung eines Medikamentes aus einer Spritze nach dem Oberbegriff des Anspruchs 1. Die Injektionsvorrichtung weist hierzu entweder ein Gehäuse auf, in dem eine Spritze fest aufgenommen ist, wobei das Gehäuse insbesondere nach einmaligem Gebrauch der Spritze zusammen mit dieser entsorgt werden kann. Oder das Gehäuse ist derart ausgebildet, dass die Spritze daran so aufgenommen werden kann, dass sie nach ihrem Gebrauch entnommen und später durch eine neue Spritze ersetzt werden kann. In jedem Fall weist die Spritze eine Injektionsnadel, eine Aufnahmekammer für das jeweilige Medikament, einen Kolben und einen Kolbenstößel auf. Die Injektionsvorrichtung weist zudem eine Steuerungsanordnung auf, mittels der die Spritze während eines Anwendungsablaufes, der wenigstens einen Einstechhub, einen Injektionshub und einen Rückholhub umfasst, gesteuert betätigt werden kann. Dabei weist die Steuerungsanordnung ein Betätigungselement auf, das durch eine manuelle, elektrische oder von einem mechanischen Kraftspeicher generierte Antriebskraft beaufschlagt werden kann. Zudem weist die Steuerungsanordnung eine gegenüber dem Gehäuse verschiebbare Spritzenaufnahme, an der die Aufnahmekammer der Spritze festlegbar ist, und eine gegenüber dem Gehäuse verschiebbare Stößelaufnahme auf, an der der Kolbenstößel der Spritze festgelegt werden kann. Ferner ist eine Übertragungseinrichtung vorgesehen, mittels der die Stößelaufnahme mit dem Betätigungselement beziehungsweise dessen Bewegung gekoppelt werden kann.

Derartige Injektionsvorrichtungen ermöglichen eine besonders einfache Verabreichung von Medikamenten zur Behandlung einer Vielzahl von Erkrankungen mittels einer Spritze, wobei die Verabreichung insbesondere von den Patienten selbst vorgenommen werden kann. Beispiele für solche Krankheiten und Anwendungsfälle sind die Thrombosevorbeugung, Zuckerkrankheit, Krebstherapie, Arthritis, Multiple Sklerose, u.s.w. Hierbei werden die Medikamente in der Regel subkutan, das heißt in das Unterhautfettgewebe verabreicht.

Auf dem Markt ist eine Vielzahl von sogenannten Sicherheitsspritzen erhältlich, die der Patient selbst bedienen und nach Gebrauch mit einer Nadelabdeckung auch sicher entsorgen kann. Eine derartige Sicherheitsspritze ist beispielsweise aus US 8,603,043 B2 zu entnehmen.

Nachteilig bei den bekannten Einweg-Spritzen ist jedoch, dass die Nadel vor der Injektion sichtbar ist und somit auch eine Verletzungsgefahr besteht. Zudem ist es für Personen mit einer Nadelphobie schwierig, eine solche Spritze zu verwenden. Bei einigen bekannten Einweg-Spritzen besteht ferner der Nachteil, dass der Patient nach einer durchgeführten Injektion die Nadel beziehungsweise die Spritze selbst zurückziehen muss und der Nadelschutz erst danach angebracht werden kann. Auch liegt die für viele Anwendungen erforderliche Verweilzeit allein im Ermessen des Patienten, was wiederum dazu führt, dass bei vielen Anwendungen keine ausreichende Verweilzeit eingehalten wird, während der die Nadel noch im Gewebe verbleibt. Hierdurch kann es passieren, dass der durch das injizierte Medikament aufgebaute Flüssigkeitsdruck nicht ausreichend abgebaut wird und es zu einem Austritt des Medikaments am Einstechkanal kommt.

Aus DE 203 11 996 U1 ist wiederum eine vollautomatische Injektionsvorrichtung bekannt, mittels der die Spritze während eines Anwendungsablaufes, der einen Einstechhub, einen Injektionshub, eine Verweilzeit und einen Rückholhub umfasst, gesteuert betätigt werden kann und bei der die oben genannten Nachteile somit nicht auftreten. Die hierbei verwendete Steuerungsanordnung erfordert dabei jedoch eine gewisse Komplexität, die keine wirtschaftliche Verwendung der Injektionsvorrichtung als Einweg-Injektionsvorrichtung erlaubt.

FR 2 767 479 A1 offenbart eine Injektionsvorrichtung mit einem Gehäuse, an dem ein zweistufiges Zahnrad verdrehbar gelagert ist Eine größere Verzahnung des Zahnrades kämmt dabei mit einem Kolbenstößel, der an einem Kolben einer Spritze anliegt, mittels dem ein in der Spritze aufgenommenes Medikament injiziert werden kann. Gleichzeitig kämmt eine kleinere Verzahnung des Zahnrades mit einem schieberförmigen Betätigungselement, das außerhalb des Gehäuses mit einer Zug- oder einer Druckkraft beaufschlagt werden kann, um das Zahnrad über die kleine Verzahnung in eine Drehbewegung zu versetzen und dadurch den Kolbenstößel über die große Verzahnung gegenüber dem Gehäuse zu verlagern.

Die Aufgabe der Erfindung ist es, bei einer gattungsgemäßen Injektionsvorrichtung die genannten Nachteile zu vermeiden und eine sichere und bedienerfreundliche Verwendung bei kostengünstiger Herstellung zu ermöglichen.

Diese Aufgabe wird durch eine Injektionsvorrichtung mit den Merkmalen des Anspruchs 1 gelöst. Dabei weist die Übertragungseinrichtung ein Zahnradgetriebe auf, mittels dem die Stößelaufnahme in Abhängigkeit von einer Relativbewegung des Betätigungselementes gegenüber dem Gehäuse angetrieben werden kann. Hierzu weist das Zahnradgetriebe am Gehäuse selbst eine Verzahnung auf, die beispielsweise einteilig mit dem Gehäuse ausgeformt oder an diesem fest gelagert ist. Die Verwendung eines Zahnradgetriebes als wesentlicher Bestandteil der Übertragungseinrichtung ermöglicht es hierbei, die Steuerungsanordnung mit einer sehr geringen Anzahl von Bauteilen umzusetzen. Zudem können diese für die Übertragungseinrichtung beziehungsweise die Steuerungsanordnung benötigten Bauteile zumindest größtenteils vorzugsweise jedoch vollständig aus einem kostengünstigen Material, wie insbesondere Kunststoff hergestellt werden. Durch die Verwendung des Zahnradgetriebes kann somit einerseits ein hoher Bedienungskomfort und eine genaue Steuerungsfunktion gewährleistet werden während die Injektionsvorrichtung relativ kostengünstig hergestellte werden kann, was wiederum die Verwendung der Injektionsvorrichtung als Einwegprodukt ermöglicht.

In einer besonders vorteilhaften Ausführungsform weist das Zahnradgetriebe eine erste betätigungselementseitige Verzahnung auf, die in Antriebsrichtung des Betätigungselementes mit einer ersten gehäuseseitigen Verzahnung unter Erzeugung einer ersten Drehrichtung in kämmenden Eingriff gebracht werden kann. Beabstandet dazu kann die erste betätigungselementseitige Verzahnung zudem mit einer auf einer anderen Seite des Gehäuses angeordneten zweiten gehäuseseitigen Verzahnung unter Erzeugung einer zweiten Drehrichtung in kämmenden Eingriff gebracht werden, wobei die zweite Drehrichtung entgegengesetzt zur ersten Drehrichtung gerichtet ist. Auf diese Weise ist es möglich, mit Aufbringung der Antriebskraft am Betätigungselement in nur eine einzige Beaufschlagungsrichtung mittels der Übertragungseinrichtung zwei zueinander entgegen gesetzte Bewegungen an der Spritzenaufnahme und/oder der Stößelaufnahme zu generieren. Auf diese Weise ist eine besonders einfache Umsetzung eines Einstech- beziehungsweise Injektionshubes gegenüber einem Rückholhub möglich.

Dabei ist es günstig, wenn das Zahnradgetriebe ein am Betätigungselement verdrehbar gelagertes Zahnrad aufweist, das sowohl mit einer gehäuseseitigen Verzahnung als auch mit einer stößelaufnahmeseitigen Verzahnung in kämmenden Eingriff bringbar ist. Hierdurch kann die Antriebskraft in besonders einfacher Weise in Abhängigkeit der Relativbewegung des Betätigungselementes gegenüber dem Gehäuse vom Betätigungselement auf die Stößelaufnahme übertragen werden.

Vorteilhafterweise kann das Zahnrad zusätzlich mit einer spritzenaufnahmeseitigen Verzahnung wenigstens in kämmenden Eingriff gebracht werden. Hierdurch ist es möglich, mittels der Antriebskraft gleichzeitig die Spritzenaufnahme und die Stößelaufnahme anzutreiben und somit auch diese in vorgegebener Relation zueinander zu bewegen.

Zudem ist es günstig, wenn an dem Zahnrad neben der ersten betätigungselementeseitige Verzahnung wenigstens eine weitere zweite betätigungselementseitige Verzahnung vorgesehen ist und die erste betätigungselementseitige Verzahnung eine erste Zähnezahl und die zweite betätigungselementseitige Verzahnung eine zweite Zähnezahl aufweist, die unterschiedlich zur ersten Zähnezahl ist. Durch diese wenigstens zwei unterschiedlichen Zähnezahlen ist es möglich, über unterschiedliche Bewegungsabschnitte des Betätigungselementes hinweg, unterschiedliche Relativgeschwindigkeiten zwischen einem oder mehreren verschiebbaren Bauteilen und dem Gehäuse zu generieren. Dabei können die beiden betätigungselementseitigen Verzahnungen in Antriebsrichtung des Betätigungselementes mit zwei Gegenverzahnungen unter Erzeugung einer Drehbewegung mit unterschiedlichen Übersetzungen in kämmenden Eingriff gebracht werden. Auf diese Weise ist die Erzeugung einer gleichzeitigen und untereinander in einer bestimmten Relation zueinander stehenden Bewegung zweier verschiebbarer Teile gegenüber dem Gehäuse möglich.

Ferner ist es günstig, wenn die Verzahnungen und Gegenverzahnungen wenigstens teilweise als Spitzverzahnungen ausgebildet sind, wodurch eine Blockierung der Übertragungseinrichtung durch genau aufeinander zu stehen kommende Zahnkämme verhindert werden kann. In einer weiteren vorteilhaften Ausführungsform sind die Verzahnungen und Gegenverzahnungen wenigstens teilweise federnd ausgebildet. Auf diese Weise kann eine vollständige Blockierung der Steuerungsanordnung durch genau aufeinander zu stehen kommende Zahnkämme gänzlich ausgeschlossen werden, da in diesem Fall entweder das am Betätigungselement gehaltene Zahnrad oder die jeweilige Gegenverzahnung sich soweit elastische verlagert, bis die Blockierung aufgehoben und ein kämmender Eingriff hergestellt ist. Ferner ist es günstig, wenn die gehäuseseitigen Verzahnungen in Beaufschlagungsrichtung wenigstens bereichsweise ansteigende Zahnhöhen aufweisen. Hierdurch können die betätigungselementeseitigen Verzahnungen besonders gleichmäßig und störungsfrei mit den jeweiligen Gegenverzahnungen in Eingriff gebracht werden.

Vorteilhafterweise sind an der Stößelaufnahme Ferderelemente vorgesehen, mittels denen am Kolbenstößel eine Vorspannung in Antriebsrichtung angelegt werden kann. Durch diese Vorspannung können an der Spritzenaufnahme oder an der Stößelaufnahme Fertigungstoleranzen des Gehäuses, der Spritze und/oder Toleranzen hinsichtlich des Füllstandes der Spritze ausgeglichen werden.

Zudem ist es günstig, wenn wenigstens ein mit dem Betätigungselement zusammenwirkendes Dämpfungselement vorgesehen ist, wodurch insbesondere bei einer manuellen Beaufschlagung des Betätigungselementes eine gleichmäßige Bewegung derselben und somit der Steuerungsanordnung insgesamt gewährleistet werden kann.

Zudem ist vorteilhafterweise ein Blockierungsmechanismus vorgesehen, mittels dem das Betätigungselement in einer Ausgangsstellung bis zum Erreichen eines Schwellenwertes einer in Antriebsrichtung wirkenden Antriebskraft blockiert werden kann. Hierdurch wird sichergestellt, dass der Anwendungsablauf erst dann startet, wenn die bedienende Person das Betätigungselement mit einer ausreichenden Antriebskraft beaufschlagt. Auf diese Weise wird auch gewährleistet, dass der Anwendungsablauf mit einer ausreichenden Geschwindigkeit abläuft.

Von Vorteil ist es dabei, wenn der Blockierungsmechanismus eine an einen Anschlag anlegbare Federnase aufweist, wodurch die Blockierung des Betätigungselementes bis zum Schwellenwert besonders einfach eingerichtet werden kann. In einer alternativen Ausführungsform weist der Blockierungsmechanismus ein abscherbares Verriegelungselement auf, wodurch der Blockierungsmechanismus besonders kostengünstig hergestellt werden kann. In einer weiteren alternativen Ausführungsform weist der Blockierungsmechanismus ein gegen eine Federkraft verschwenkbares Verriegelungselement auf. Hierdurch kann die Injektionsvorrichtung durch Auswahl entsprechender Federelemente besonders einfach auf unterschiedliche Schwellenwerte der Antriebskraft eingestellt werden. In einer weiteren alternativen Ausführungsform weist der Blockierungsmechanismus ein mit dem Betätigungselement zusammen wirkendes schaltbares Zahnrad auf, wodurch eine Antriebsgeschwindigkeit des Betätigungselementes bei Überschreitung des Schwellenwertes der Antriebskraft besonders genau festgelegt werden kann. In einer besonders vorteilhaften Ausführungsform kann die Spritzenaufnahme zur Durchführung des Einstechhubes fest mit der Stößelaufnahme gekoppelt werden. Hierdurch kann eine störende Relativbewegung der Stößelaufnahme gegenüber der Spritzenaufnahme während des Einstechhubes ausgeschlossen werden. Dabei ist es besonders günstig, wenn die Kopplung mittels der ersten betätigungselementeseitigen Verzahnung des Zahnrades hergestellt werden kann, das hierzu gleichzeitig mit jeweils einer Gegenverzahnung der Spritzenaufnahme und der Stößelaufnahme in Eingriff steht. Bei dieser Vorgehensweise sind für die Kopplung beider Aufnahmen keine gesonderten Bauteile erforderlich. Vorteilhafterweise ist das Zahnrad zur Aufhebung der Kopplung zwischen der Spritzenaufnahme und der Stößelaufnahme nach Abschluss des Einstechhubes verlagerbar, wodurch eine besonders einfache Entkopplung möglich ist. Dabei ist es günstig, wenn das Zahnrad verschwenkbar gehalten ist, so dass der Zeitpunkt der Entkopplung besonders einfach festlegbar ist, wie beispielsweise durch gegenüber dem Gehäuse fest angebrachte Auslenkmittel. Alternativ hierzu ist es günstig, wenn das Zahnrad in radialer Richtung verlagerbar gehalten ist, wodurch die vollständige Entkopplung besonders schnell vorgenommen werden kann. In einer weiteren alternativen Ausführungsform ist die spritzenaufnahmeseitige Verzahnung zur Aufhebung der Kopplung zwischen der Spritzenaufnahme und der Stößelaufnahme nach Abschluss des Einstechhubes verlagerbar, wodurch das am Betätigungselement gehaltene Zahnrad besonders stabil gelagert werden kann, während die Entkopplung allein durch Beabstandung der spritzenaufnahmeseitigen Verzahnung erfolgt.

In einer weiteren besonders vorteilhaften Ausführungsform kann die Kopplung mittels eines in eine Aufnahme lösbar eingreifenden Eingriffselementes hergestellt werden. Hierdurch kann die Kopplung mit einfachen Mitteln auch unabhängig vom kämmenden Eingriff des Zahnrades vorgenommen werden. Alternativ hierzu ist die Kopplung mittels eines verlagerbaren Kulissensteins herstellbar, was eine besonders kostengünstige Umsetzung der Kopplung und Entkopplung ermöglicht. In einer weiteren alternativen Ausführungsform ist die Kopplung mittels eines abscherbaren Verriegelungselementes herstellbar, was eine besonders einfache Entkopplung ermöglicht. In einer weiteren alternativen Ausführungsform ist die Kopplung mittels eines schaltbaren Freilaufes herstellbar, wodurch die Entkopplung ohne einen seitens des Bedieners merkbaren Kraftaufwand erfolgen kann.

Besonders vorteilhaft ist es, wenn am Zahnradgetriebe ein Leerhub zur Erzeugung einer Verweilzeit vorgesehen ist, über den hinweg trotz Relativbewegung des Betätigungselementes gegenüber dem Gehäuse keine Antriebskraft auf die Stößelaufnahme übertragbar ist. Hierdurch ist auch bei Verwendung des Zahnrades als wesentlicher Teile der Übertragungseinrichtung auch die für viele Anwendungen erforderliche Verweilzeit vorab festlegbar, während der die Nadel noch im Gewebe verbleibt. Hierdurch kann sichergestellt werden, dass der durch das injizierte Medikament aufgebaute Flüssigkeitsdruck vor dem Entfernen der Spritze ausreichend abgebaut wird und es zu keinem wesentlichen Austritt des Medikaments am Einstechkanal kommt.

Zudem ist es günstig, wenn die zweite betätigungselementseitige Verzahnung des Zahnrades zur Durchführung des Rückholhubes mit der zweiten Gegenverzahnung in kämmenden Eingriff bringbar ist, die durch die gehäuseseitige Verzahnung gebildet ist, und die erste Verzahnung des Zahnrades dabei gleichzeitig mit der stößelaufnahmeseitigen Verzahnung im kämmenden Eingriff steht, wobei zudem die Spritzenaufnahme fest mit der Stößelaufnahme koppelbar ist, Hierdurch ist ein insbesondere hinsichtlich der Verweilzeit zeitlich genau abgestimmter Start des Rückholhubes einrichtbar.

Vorteilhafterweise kann die Kopplung dabei mittels der ersten Verzahnung des Zahnrades hergestellt werden, wodurch wiederum keine zusätzlichen Elemente für die Kopplung der Stößelaufnahme mit der Spritzenaufnahme benötigt werden. Alternativ hierzu kann die Kopplung mittels eines in eine Aufnahme lösbar eingreifenden Federelementes hergestellt werden, wodurch die Kopplung zwischen Stößelaufnahme und Spritzenaufnahme auch unabhängig vom Zahnradgetriebe erfolgen kann.

In einer vorteilhaften Ausführungsform ist das Betätigungselement zudem in einer Endstellung, in der der Rückholhub abgeschlossen und die Nadel wieder vollständig innerhalb des Gehäuses angeordnet ist, verriegelbar. Hierdurch kann eine insbesondere unbeabsichtigte neuerliche Betätigung der Injektionsvorrichtung verhindert werden, bei der die Nadel wieder aus dem Gehäuse austreten und somit zu Verletzungen führen könnte. Auf diese Weise ist nach einer erfolgten Verwendung der Injektionsvorrichtung eine sichere Entsorgung derselben möglich. Die Verriegelung erfolgt dabei vorteilhafterweise mittels einer am Betätigungselement gehaltenen Rastnase, die in der Endstellung an einen Rastanschlag anlegbar ist. Hierdurch sind die zur Herstellung der Verriegelung benötigen Elemente besonders einfach und kostengünstig herstellbar.

In einer besonders vorteilhaften Ausführungsform weist das Gehäuse einen stirnseitigen Gehäuseteil auf, der zur Einstellung einer Einstechtiefe gegenüber dem übrigen Gehäuse verlagerbar ist. Hierdurch kann die Injektionsvorrichtung mit einfachen Mitteln auf verschiedene Anwendungsformen eingestellt werden. Dabei ist es günstig, wenn der stirnseitige Gehäuseteil verschiebbar am übrigen Gehäuse gehalten ist, wodurch eine schnelle Einstellung der Einstechtiefe über einen relativ großen Stellbereich möglich ist. Alternativ hierzu ist der stirnseitige Gehäuseteil über eine verstellbare Schraubverbindung am übrigen Gehäuse gehalten, wodurch eine stabile und stufenlose Einstellung der jeweils benötigten Einstechtiefe möglich ist. In einer weiteren alternativen Ausführungsform ist der stirnseitige Gehäuseteil über eine Steuerkurve gegenüber dem übrigen Gehäuse verlagerbar, wodurch insbesondere verschiedene vorbestimmte Einstechtiefen besonders einfach eingestellt werden können. In einer weiteren alternativen Ausführungsform ist der stirnseitige Gehäuseteil mittels eines verlagerbaren Betätigungselementes verstellbar, was eine besonders komfortable Einstellung verschiedener Einstechtiefen ermöglicht. In einer weiteren alternativen Ausführungsform ist der stirnseitige Gehäuseteil durch jeweils einen von mehreren austauschbaren Abstandshaltern gebildet, der am übrigen Gehäuse gelagert ist. Durch Anbringung jeweils eines dieser Abstandshalter kann an der Injektionsvorrichtung somit eine bestimmte Einstechtiefe eingestellt werden.

Besonders vorteilhaft ist es zudem, wenn das Betätigungselement über einen mechanischen Kraftspeicher mit der Antriebskraft beaufschlagbar ist. Auf diese Weise kann die Injektionsvorrichtung auch von Patienten genutzt werden, die motorisch eingeschränkt sind und somit Probleme haben, das Betätigungselement während der Verwendung der Injektionsvorrichtung mit der notwendigen Antriebskraft zu beaufschlagen. Dabei ist es günstig, wenn der mechanische Kraftspeicher eine Konstantkraftfeder aufweist, wodurch der mechanische Kraftspeicher eine besonders gleichmäßige Antriebskraft erzeugen kann.

In den Figuren ist eine beispielhafte Ausführungsform der Erfindung dargestellt, Es zeigen:
- Figur 1: eine perspektivische Explosionsansicht einer erfindungsgemäßen Injektionsvorrichtung,
- Figur 2: eine perspektivische Ansicht der Injektionsvorrichtung in einer Ausgangsstellung,
- Figur 3: eine perspektivische Ansicht einer Steuerungsanordnung der Injektionsvorrichtung in der Ausgangsstellung,
- Figur 4: eine perspektivische Ansicht der Injektionsvorrichtung während eines Einstechhubes,
- Figur 5: eine perspektivische Ansicht der Steuerungsanordnung während des Einstechhubes,
- Figur 6: eine perspektivische Ansicht der Steuerungsanordnung während eines Injektionshubes,
- Figur 7: eine perspektivische Ansicht der Steuerungsanordnung zu Beginn eines Leerhubes,
- Figur 8: eine perspektivische Ansicht der Steuerungsanordnung während des Leerhubes,
- Figur 9: eine perspektivische Ansicht der Steuerungsanordnung zu Beginn eines Rückholhubes,
- Figur 10: eine perspektivische Ansicht der Steuerungsanordnung während des Rückholhubes,
- Figur 11: eine perspektivische Ansicht der Steuerungsanordnung in einer Endstellung,
- Figur 12: eine perspektivische Ansicht der Injektionsvorrichtung in der Endstellung,
- Figur 13: eine perspektivische Ansicht einer Steuerungsanordnung einer alternativen Ausführungsform der Injektionsvorrichtung in einer Ausgangsstellung,
- Figur 14: eine perspektivische Ansicht der alternativen Ausführungsform der Injektionsvorrichtung in der Ausgangsstellung,
- Figur 15: eine perspektivische Explosionsansicht der Injektionsvorrichtung gemäß Fig. 1 mit einer Einstechtiefeneinstellung,
- Figur 16: eine perspektivische Ansicht der Einstechtiefeneinstellung nach Fig. 15 in einer ersten Stellposition,
- Figur 17: eine perspektivische Ansicht der Einstechtiefeneinstellung nach Fig. 15 in einer zweiten Stellposition und
- Figur 18: eine perspektivische Ansicht einer weiteren alternativen Ausführungsform der Injektionsvorrichtung mit einem mechanischen Kraftspeicher.

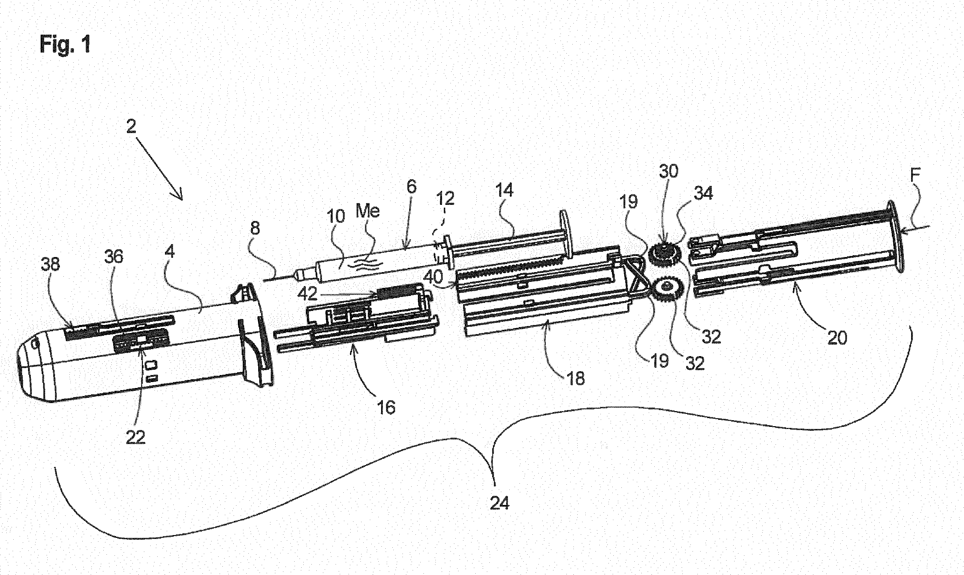

Fig. 1 zeigt die einzelnen Elemente einer Injektionsvorrichtung 2 mit einem Gehäuse 4, in dem eine Spritze 6 fest aufgenommen ist, die eine Injektionsnadel 8, eine Aufnahmekammer 10, einen Kolben 12 und einen Kolbenstößel 14 aufweist. Hierzu sind in dem Gehäuse 4 eine Spritzenaufnahme 16, an der die Aufnahmekammer 10 festlegbar ist, und eine Stößelaufnahme 18, an der der Kolbenstößel 14 festlegbar ist, verschiebbar aufgenommen. Mittels zweier an einem Ende der Stößelaufnahme 18 vorgesehener Federelemente 19 kann beim Einbau der Spritze 6 eine gewisse Vorspannung erzeugt werden, mittels der gewisse Fertigungstoleranzen oder unterschiedliche Füllstände ausgeglichen werden können.

Die Spritzenaufnahme 16 und die Stößelaufnahme 18 bilden dabei zusammen mit einem Betätigungselement 20, das von außen insbesondere manuell mit einer Antriebskraft F beaufschlagt werden kann, und mit einer Innenseite 22 des Gehäuses 4 eine Steuerungsanordnung 24, mittels der die Spritze 6 während eines Anwendungsablaufes zur Verabreichung eines in der Aufnahmekammer 10 aufgenommenen Medikamentes Me in gesteuerter Weise betätigt wird. Dabei umfasst der vorgesehene Anwendungsablauf einen Einstechhub, während dessen die Nadel 8 in ein Gewebe des jeweiligen Patienten eingestochen wird, einen Injektionshub, während dessen das Medikament Me injiziert wird, einen Leerhub, während dessen ein Überdruck der injizierten Flüssigkeit abgebaut werden kann, sowie einen Rückholhub, durch den die Nadel 8 wieder in das Innere des Gehäuses 4 verlagert wird.

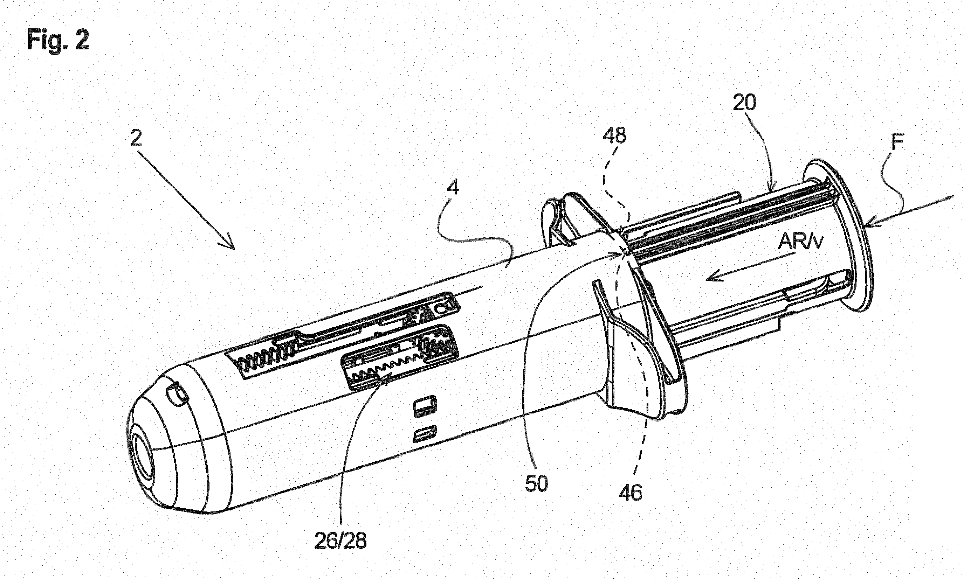

Zur gesteuerten Durchführung dieses Anwendungsablaufes weist die Steuerungsanordnung 24 eine Übertragungseinrichtung 26 auf, die im Wesentlichen durch ein Zahnradgetriebe 28 gebildet ist, wie es beispielsweise aus Fig. 2 zu entnehmen ist.

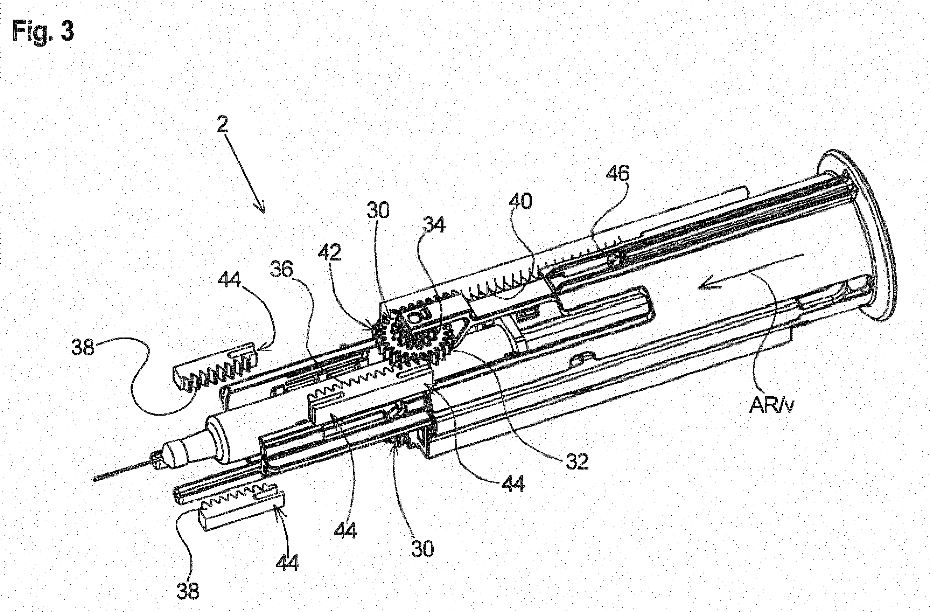

Wie insbesondere aus Fig. 3 zu entnehmen ist, weist das Zahnradgetriebe 28 zwei an dem Betätigungselement 20 verdrehbar gelagerte zweistufige Zahnräder 30 auf, die jeweils eine erste betätigungselementeseitige Verzahnung 32 mit einer ersten Zähnezahl und eine zweite betätigungselementeseitige Verzahnung 34 mit einer zweiten Zähnezahl bilden.

Entlang einer Antriebsrichtung AR des Betätigungselementes 20 können diese Zahnräder 30 über den Anwendungsablauf hinweg mit verschiedenen Gegenverzahnungen in kämmenden Eingriff gebracht werden. Diese Gegenverzahnungen sind dabei durch eine erste gehäuseseitige Verzahnung 36 und eine zu dieser beabstandeten und auf einer gegenüber liegenden Seite der Innenseite 22 des Gehäuses 4 angeordnete zweite gehäuseseitige Verzahnung 38 sowie durch eine stößelaufnahmeseitige Verzahnung 40 und eine spritzenaufnahmeseitige Verzahnung 42 gebildet. Zur leichteren Darstellung sind hierbei die gehäuseseitigen Verzahnungen 36, 38 ohne das übrige Gehäuse 4 wiedergegeben.

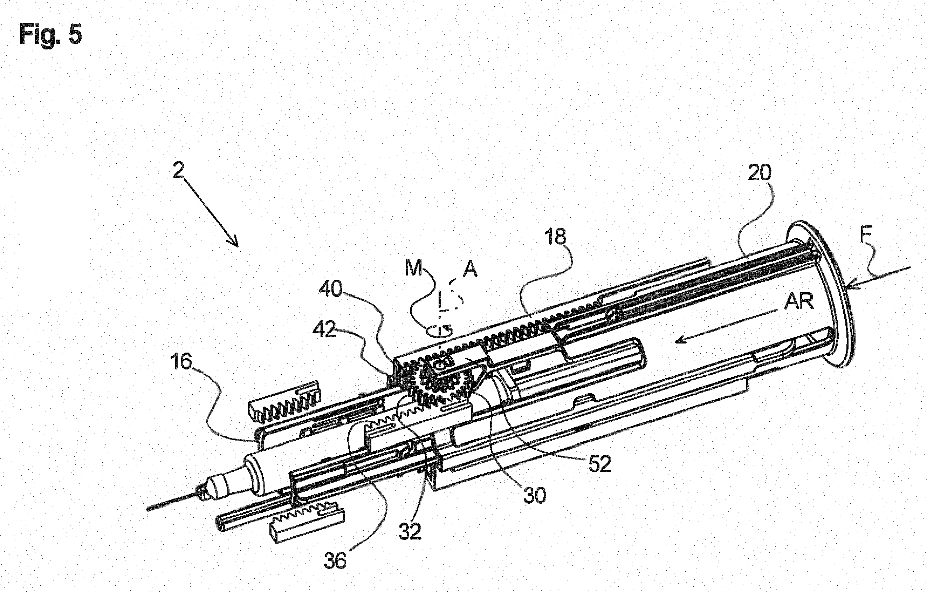

Um die Zahnräder 30 während eines Anwendungsablaufes leichter mit den verschiedenen Gegenverzahnungen in kämmenden Eingriff bringen zu können, können die einzelnen Verzahnungen als Spitzverzahnungen ausgebildet sein. Zudem ist es günstig, wenn die Verzahnungen wenigstens teilweise federnd ausgebildet sind, wie beispielhaft durch Federabschnitte 44 an den Enden der gehäuseseitigen Verzahnungen 36, 38 dargestellt. Alternativ oder zusätzlich hierzu können die Gegenverzahnungen an den Enden, an denen die Zahnräder 30 in Antriebsrichtung R in kämmenden Eingriff kommen, ansteigende Zahnhöhen aufweisen, wie beispielsweise aus Fig. 5 zu entnehmen ist.

Nachfolgend wird der Anwendungsablauf anhand der Figuren beschrieben, wobei lediglich auf eines der Zahnräder 30 Bezug genommen wird.

Zur Verabreichung des in der Aufnahmekammer 10 aufgenommenen Medikamentes Me wird die Injektionsvorrichtung 2 zunächst mit dem stirnseitigen Ende des Gehäuses 4 an der betreffenden Stelle des Körpers des jeweiligen Patienten positioniert, an der das Medikament Me injiziert werden soll. Die Aktivierung der Injektionsvorrichtung 2 erfolgt dann durch manuelle Kraftbeaufschlagung des Betätigungselementes 20 mit der Antriebskraft F, wie beispielsweise durch einen Daumen des Patienten (nicht dargestellt).

Die Figuren 2 und 3 zeigen die Injektionsvorrichtung 2 in einer betriebsbereiten Ausgangsstellung, in der die Beaufschlagung mit der Antriebskraft F erfolgt. Hierbei liegt eine Federnase 46, die am Betätigungselement 20 elastisch verlagerbar gehalten ist, an einen durch einen Rand gebildeten Anschlag 48 des Gehäuses 4 an, wie insbesondere in Fig. 2 durch gestrichelte Linien dargestellt. Die Federnase 46 und der Anschlag 48 bilden hierdurch einen Blockiermechanismus 50 mittels dem ein Schwellenwert definiert ist, der für die Antriebskraft F in Antriebsrichtung AR erreicht werden muss, um den Anwendungsablauf beziehungsweise den Einstechhub zu starten. Dabei wird bei Erreichen des Schwellenwertes die Federnase 46 elastische nach innen verlagert, so dass sich das Betätigungselement 20 in Antriebsrichtung AR verlagern kann, um den Einstechhub auszuführen.

Alternativ zu der dargestellten Anordnung aus Federnase 46 und Anschlag 48 kann der Blockiermechanismus 50 auch durch jeden anderen bekannten und geeigneten Mechanismus gebildet sein, durch den sich ein entsprechender Schwellenwert der Antriebskraft F definieren lässt, wie beispielsweise durch eine Anordnung mit einem abscherbaren Verriegelungselement, mit einem gegen eine Federkraft verschwenkbaren Verriegelungselement, mit einem mit dem Betätigungselement 20 zusammen wirkenden schaltbaren Zahnrad, und so weiter (nicht dargestellt).

Um bei dieser Beaufschlagung des Betätigungselementes 20 mit der Antriebskraft F eine möglichst gleichmäßige Antriebsbewegung mit einer geeigneten Antriebsgeschwindigkeit v in Antriebsrichtung AR erzeugen zu können, kann zudem wenigstens ein Dämpfungselement vorgesehen sein, das mit dem Betätigungselement 20 zusammen wirkt und hierzu entweder direkt oder indirekt mit diesem bewegungsgekoppelt ist. Das Dämpfungselement kann dabei beispielsweise durch einen Rotationsdämpfer oder durch einen Lineardämpfer gebildet sein. Darüber hinaus wäre es auch möglich, mehrere Linear- und/oder Rotationsdämpfer vorzusehen, die beispielsweise mit dem Betätigungselement 20, der Spritzenaufnahme 16 und/oder der Stößelaufnahme 20 zusammen wirken, um dadurch eine Verlagerungsgeschwindigkeit in den einzelnen Hubphasen des Anwendungsablaufes separat einstellen und vergleichmäßigen zu können (nicht dargestellt). In jedem Fall können hierfür alle bekannten und geeigneten Ausführungsformen von Linear- und Rotationsdämpfern Verwendung finden.

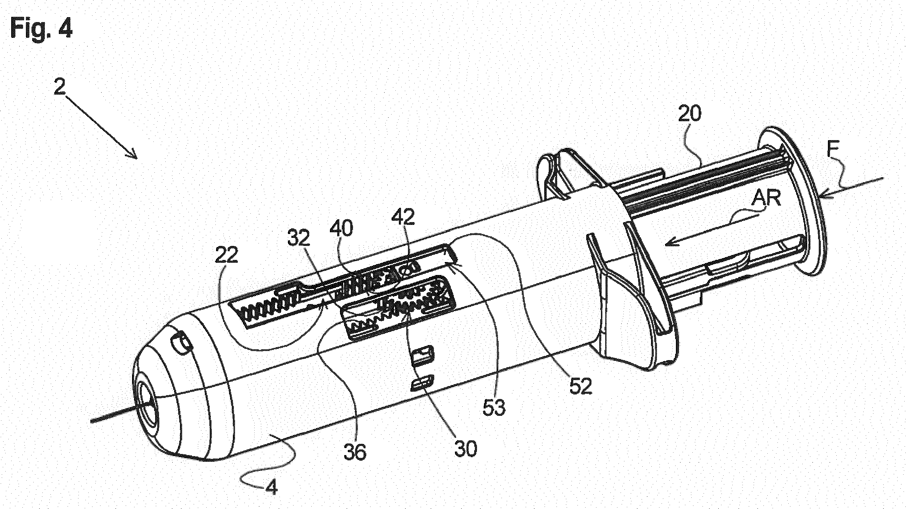

Fig.4 und Fig. 5 zeigen die Injektionsvorrichtung 2 während des Einstechhubes. Wie hieraus zu entnehmen ist, steht hierbei die erste betätigungselementeseitige Verzahnung 32 sowohl mit der ersten gehäuseseitigen Verzahnung 36 als auch mit der stößelaufnahmeseitigen Verzahnung 40 in kämmendem Eingriff. Zudem ist der die betätigungselementeseitige Verzahnung 32 bildende Teil des Zahnrades 30 so breit ausgeführt und derart positioniert, dass er benachbart zur stößelaufnahmeseitigen Verzahnung 40 auch gleichzeitig mit der spritzenaufnahmeseitigen Verzahnung 42 in kämmendem Eingriff steht. Hierdurch ist die Spritzenaufnahme 16 während des Einstechhubes über die erste betätigungselementeseitige Verzahnung 32 fest mit der Stößelaufnahme 18 gekoppelt.

Das Zahnrad 30 ist hierzu verlagerbar an einem Federarm 52 gehalten, der während des Einstechhubes an der Innenseite 22 des Gehäuses 4 anliegt und von dieser soweit nach innen gedrückt wird, dass das Zahnrad 30 sowohl mit der stößelaufnahmeseitigen Verzahnung 40 als auch mit der spritzenaufnahmeseitigen Verzahnung 42 kämmt.

Durch die Bewegung des Betätigungselementes 20 rollt somit das Zahnrad 30 auf der ersten betätigungsseitigen Verzahnung 32 an der ersten gehäuseseitigen Verzahnung 36 ab und treibt durch das dabei um eine Achse A des Zahnrades 30 entstehende Antriebsmoment M über die stößelaufnahmeseitige Verzahnung 40 und die spritzenaufnahmeseitige Verzahnung 42 die Spritzenaufnahme 16 und die Stößelaufnahme 18 gleichzeitig in Antriebsrichtung AR an, wie insbesondere in Fig. 5 dargestellt.

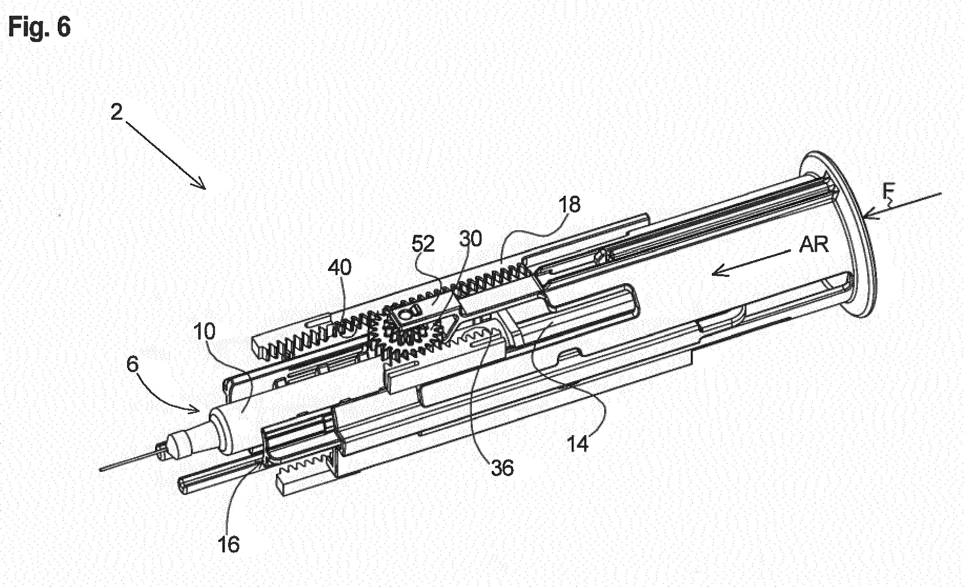

Fig. 6 zeigt die Injektionsvorrichtung 2 nach Abschluss des Einstechhubes, während des Injektionshubes. Wie hieraus zu entnehmen ist, kämmt das Zahnrad 30 zu diesem Zeitpunkt lediglich noch mit der ersten gehäuseseitigen Verzahnung 36 und der stößelaufnahmeseitigen Verzahnung 40. Auf die Spritzenaufnahme 16 erfolgt dagegen zu diesem Zeitpunkt keine Kraftübertragung vom Zahnrad 30 mehr.

Hierfür ist in das Gehäuse 4, wie insbesondere aus Fig. 4 zu entnehmen ist, eine Aussparung 53 eingelassen, in die sich der Federarm 52 soweit hinein elastisch rückverformen kann, dass das Zahnrad 30 lediglich noch mit der stößelaufnahmeseitigen Verzahnung 40 und der ersten gehäuseseitigen Verzahnung 36 kämmt. Hierdurch wird die Stößelaufnahme 18 mit dem darin aufgenommenen Kolbenstößel 14 weiter in Antriebsrichtung AR verlagert, während die Spritzenaufnahme 16 mit der darin gehaltenen übrigen Spritze 6 in der erreichten Einstechposition verbleibt.

Alternativ zu der dargestellten verschwenkbare Lagerung des Zahnrades 30 wäre hierbei auch jede andere Verlagerung zur Trennung des kämmenden Eingriffs zwischen Zahnrad 30 und spritzenaufnahmeseitiger Verzahnung 42 denkbar, wie beispielsweise eine in radialer oder axialer Richtung verstellbare Lagerung des Zahnrades 30 (nicht dargestellt).

Zudem wäre es auch denkbar zur Aufhebung der Kopplung zwischen Spritzenaufnahme 16 und Stößelaufnahme 18 nicht das Zahnrad 30 sondern die spritzenaufnahmeseitige Verzahnung 42 verlagerbar auszuführen (nicht dargestellt).

In jedem Fall wird während dieses Injektionshubes der Kolbenstößel 14 beziehungsweise der mit diesem verbundene Spritzenkolben 12 in die Aufnahmekammer 10 gepresst und dadurch das Medikament Me über die Injektionsnadel 8 in den Patienten injiziert.

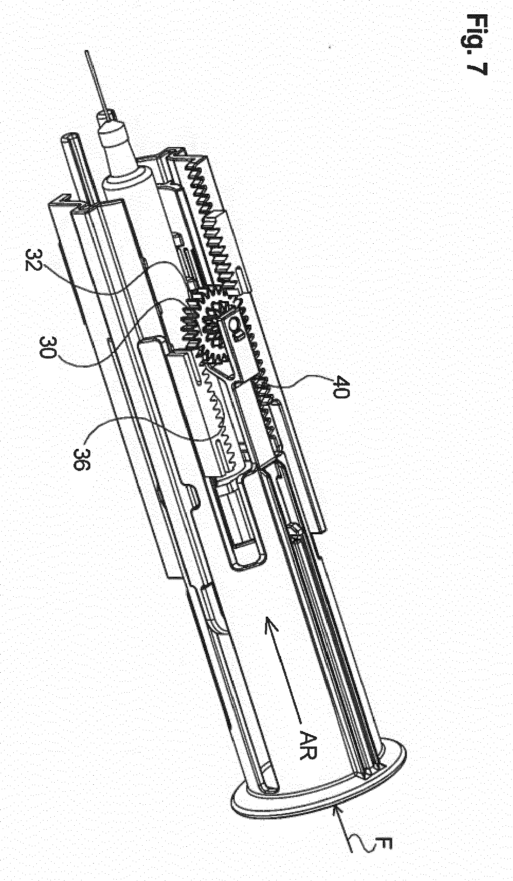

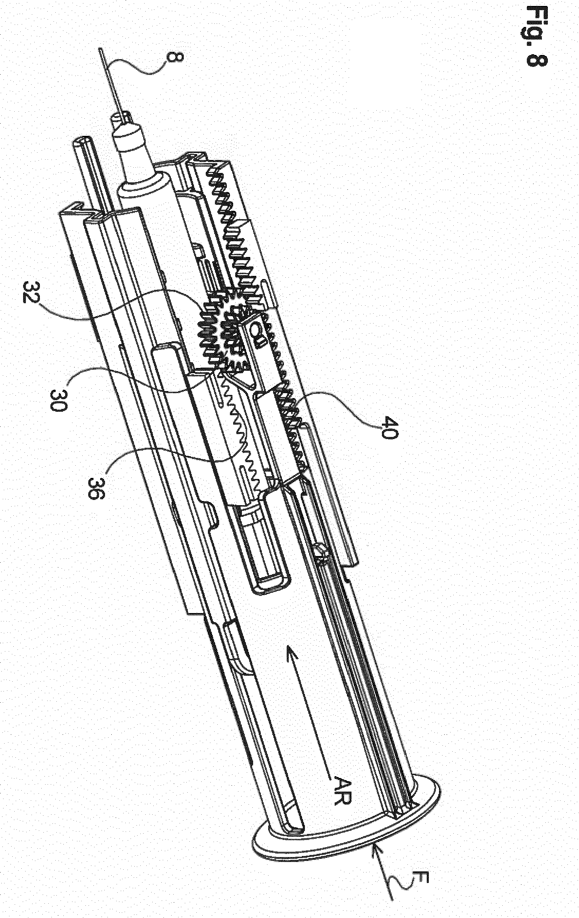

Am Ende dieses Injektionshubes gelangt das Zahnrad 30 aus dem kämmenden Eingriff mit der ersten gehäuseseitigen Verzahnung 36, wie in Fig. 7 dargestellt. Hierdurch kann das Zahnrad 30, das anschließend über seine erste betätigungselementeseitige Verzahnung 32 nur noch mit der stößelaufnahmeseitigen Verzahnung 40 kämmt, an dieser frei abrollen, ohne das es zu einer weiteren Kraftübertragung kommt, wie in Fig. 8 dargestellt. In dieser Phase des Anwendungsablaufes wird ein Leerhub erzeugt, aus dem eine Verweilzeit resultiert, während der die Injektionsnadel 8 nach abgeschlossener Injektion noch in der Haut des Patienten verbleibt, damit sich der durch das injizierte Medikament Me aufgebaute Überdruck abbauen kann, bevor die Injektionsnadel 8 aus dem Injektionskanal entfernt wird.

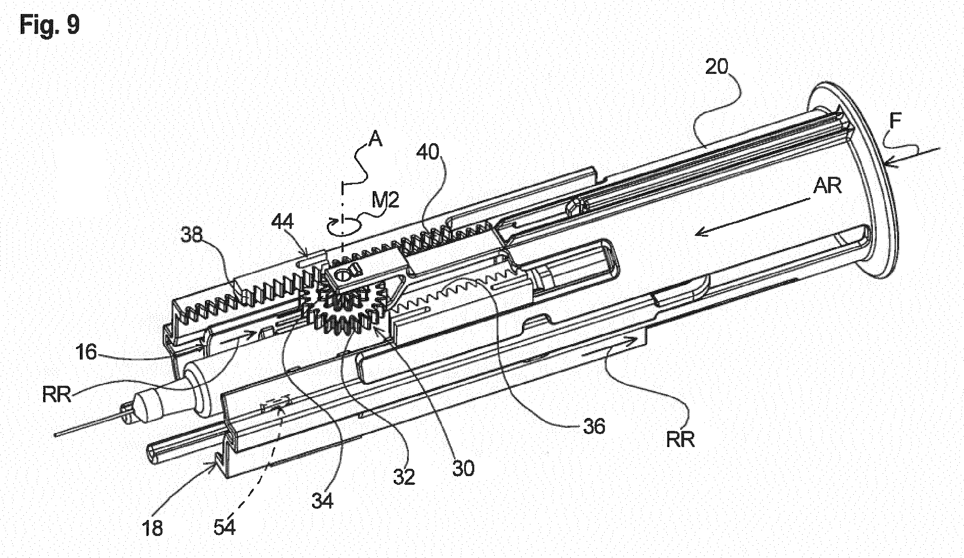

Am Ende dieses Leerhubes kommt dann die zweite betätigungselementeseitige Verzahnung 34 am Federabschnitt 44 mit der zweiten gehäuseseitigen Verzahnung 38 in kämmenden Eingriff, während die erste betätigungselementeseitige Verzahnung 32 des Zahnrades 30 weiterhin mit der stößelaufnahmeseitigen Verzahnung 40 kämmt, wie in Fig. 9 dargestellt.

Gleichzeitig wird während des Leerhubes über eine schematisch dargestellte Kopplungseinrichtung 54 eine Kopplung zwischen der Spritzenaufnahme 16 und der Stößelaufnahme 18 hergestellt. Die Kopplung kann dabei durch alle bekannten Ausführungsformen gebildet sein, die geeignet sind, die beiden Aufnahmen 16, 18 lösbar zu verbinden, wie beispielsweise durch eine Rast-anordnung mit einem in eine Aufnahme eingreifenden Federelement oder alternativ hierzu auch durch einen erneut erzeugten gleichzeitigen Eingriff des Zahnrades 30 in die stößelaufnahmeseitige Verzahnung 40 und die spritzenaufnahmeseitige Verzahnung 42 (nicht dargestellt).

Da die zweite gehäuseseitige Verzahnung 38 gegenüber der ersten gehäuseseitigen Verzahnung 36 auf der gegenüberliegenden Seite der Innenseite 22 des Gehäuses 4 ausgebildet ist, dreht sich das Zahnrad 30 in dieser Phase gegenüber dem Einstich- und Injektionshub nun in entgegen gesetzter Richtung, wobei um die Achse A auch ein entgegen gesetzt gerichtetes Moment M2 generiert wird.

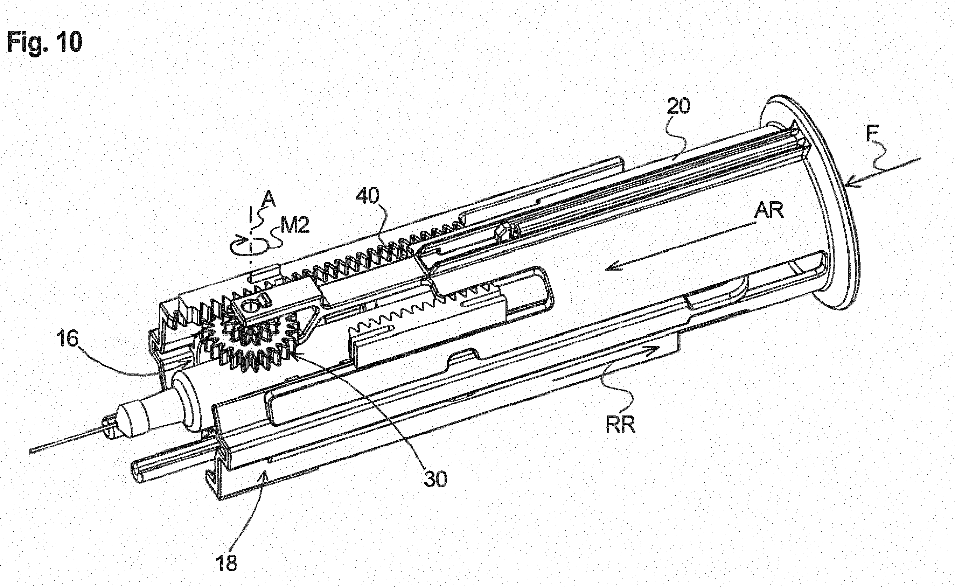

Durch diese Drehrichtungsumkehr wird die Stößelaufnahme 18, die über die stößelaufnahmeseitige Verzahnung 40 noch immer mit dem Zahnrad 30 kämmt, entgegen der Antriebsrichtung AR des Betätigungselementes 20 entlang einer Rückholrichtung RR verlagert, wie in Fig. 10 dargestellt. Aufgrund der durch die Kopplungseinrichtung 54 hergestellten Verbindung zwischen der Stößelaufnahme 18 und der Spritzenaufnahme 16 wird letztere hierbei ebenfalls entlang der Rückholrichtung RR verlagert.

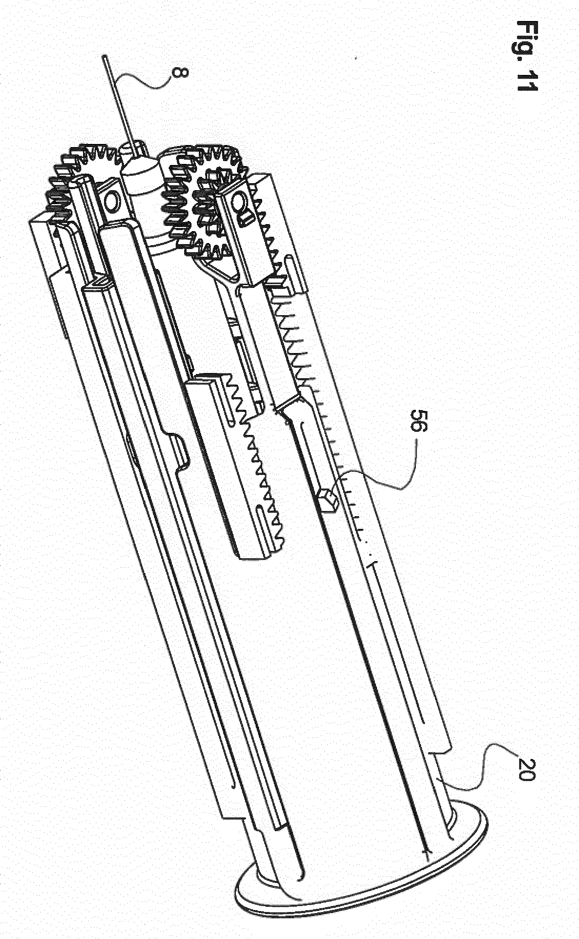

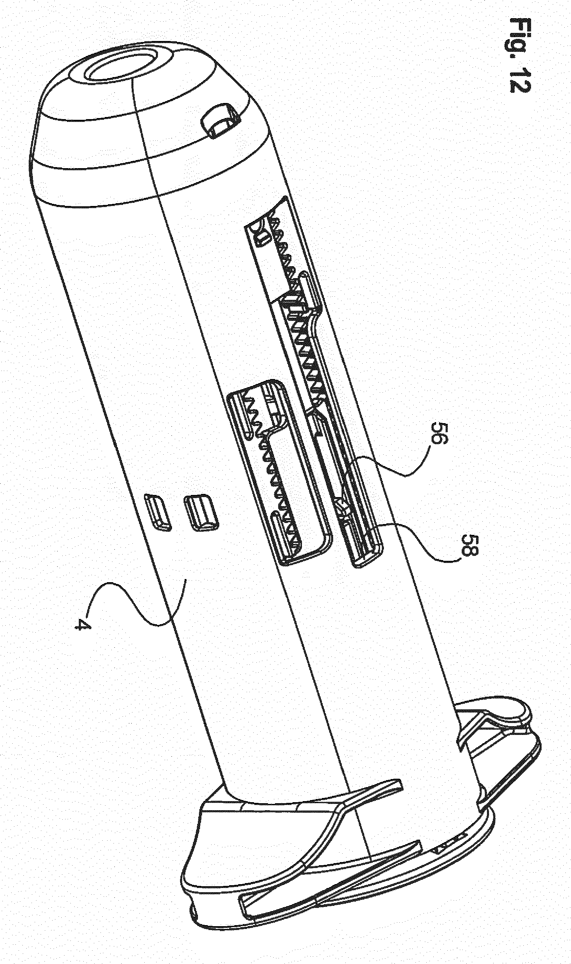

Dieser Rückholhub wird in einer Endstellung beendet, in der das Betätigungselement 20 bis zu einem Endanschlag in das Gehäuse 4 hinein verschoben ist und die Injektionsnadel 8 wider vollständig innerhalb des Gehäuses 4 angeordnet ist, wie in den Figuren 11 und 12 dargestellt. In dieser Endstellung ist das Betätigungselement 20 verriegelbar, um dessen erneutes Herausziehen aus dem Gehäuse 4 zu blockieren. Zur Verriegelung dient hierbei beispielhaft eine Rastnase 56, die an einen entsprechend positionierten Rastanschlag 58 des Gehäuses 4 anlegbar ist.

In dieser Endstellung kann die Injektionsvorrichtung 2 somit nach ihrem einmaligen Gebrauch entsorgt werden, wobei sichergestellt ist, dass die Injektionsnadel 8 nicht aus dem Gehäuse 4 heraus stehen kann.

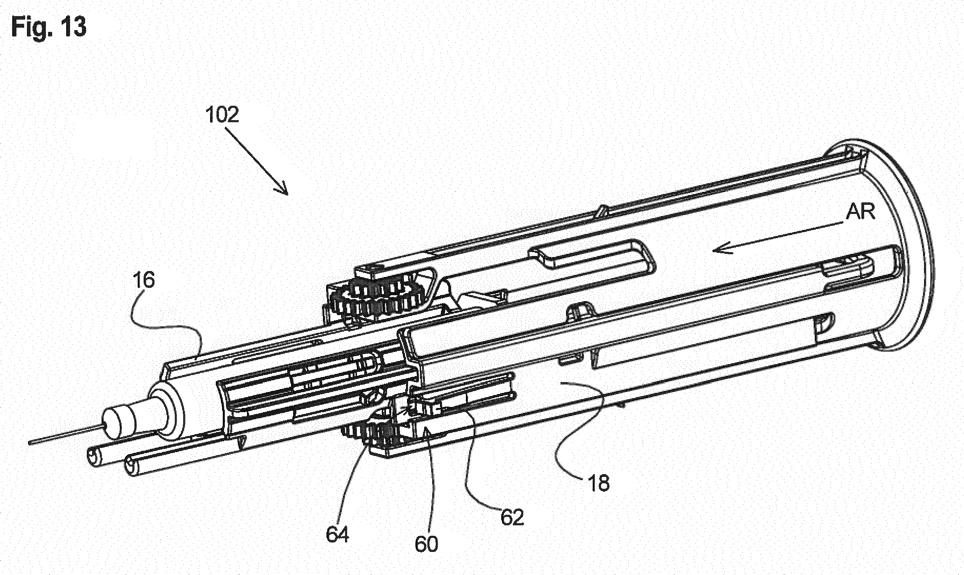

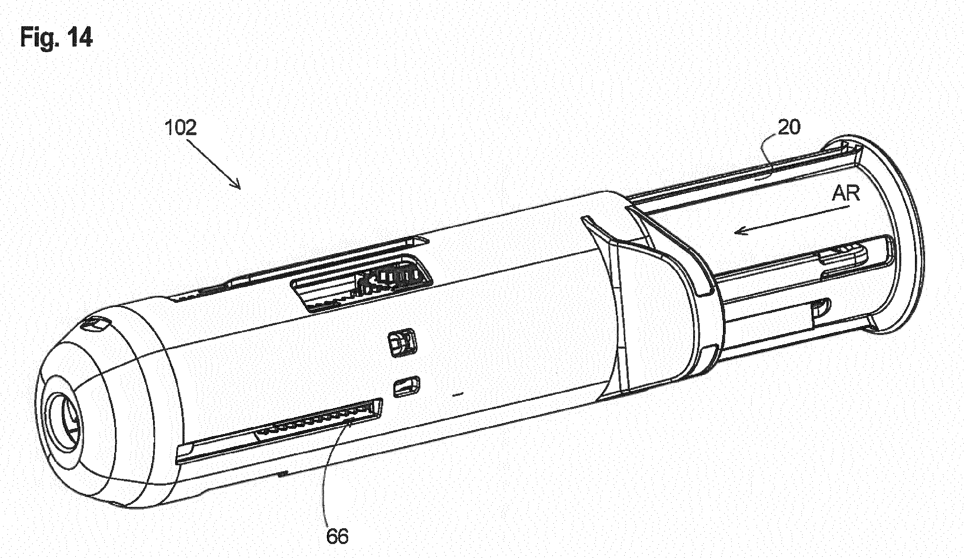

Fig. 13 und 14 zeigen eine alternative Ausführungsform der Injektionsvorrichtung 102, die ebenfalls zur Durchführung eines Anwendungsabtaufs mit einem Einstechhub, einem Injektionshub, einer Verweilzeit und einem Rückholhub dient. Die alternative Injektionsvorrichtung 102 weist gegenüber der oben beschriebenen Injektionsvorrichtung 2 einen übereinstimmenden Aufbau auf, so dass alle übereinstimmenden Elemente der alternativen Injektionsvorrichtung 102 mit übereinstimmenden Bezugszeichen gekennzeichnet sind wie bei der Injektionsvorrichtung 2.

Die alternative Injektionsvorrichtung 102 unterscheidet sich im Wesentlichen dadurch von der Injektionsvorrichtung 2, dass an der Spritzenaufnahme 16 keine spritzenaufnahmeseitige Verzahnung 42 vorgesehen ist. Zur lösbaren Bewegungskopplung der Spritzenaufnahme 16 mit der Stößelaufnahme 18 während der Durchführung des Einstechhubes ist vielmehr eine Verbindungseinrichtung 60 vorgesehen, die beispielhaft ein an der Stößelaufnahme 18 federnd gehaltenes Eingriffselement 62 aufweist, das bei der Montage der Injektionsvorrichtung 102 mit einer in die Spritzenaufnahme 16 eingelassenen Aufnahme 64 in formschlüssigen Eingriff gedrückt wird, wobei diese Eingriffsstellung in der Ausgangsstellung der Injektionsvorrichtung 102 und während des Einstechhubes durch die Innenseite 22 des Gehäuses 4 gesichert wird.

Nach Abschluss des Einstechhubes ist das durch die elastischen Rückstellkräfte nach außen gegen die Innenseite 22 drückende Eingriffselement 62 jedoch auf Höhe einer Ausnehmung 66 des Gehäuses 4 angeordnet, wie sie aus Fig. 14 zu entnehmen ist, so dass es sich aus dem formschlüssigen Eingriff mit der Spritzenaufnahme 16 lösen kann. Hierdurch kann nun die Stößelaufnahme 18 gegenüber der Spritzenaufnahme 16 weitere in Antriebsrichtung AR verlagert werden, um den Injektionshub durchzuführen.

Alternativ zu der hier dargestellten Verbindungseinrichtung 60 kann die Kopplung zwischen der Spritzenaufnahme 16 und der Stößelaufnahme 18 der Injektionsvorrichtung 102 auch durch jede sonstige bekannte und geeignete Ausführungsform der Verbindungseinrichtung 60 hergestellt werden, wie beispielsweise durch Verwendung eines verlagerbaren Kulissensteins, eines abscherbaren Verriegelungselementes oder eines schaltbaren Freilaufes (nicht dargestellt).

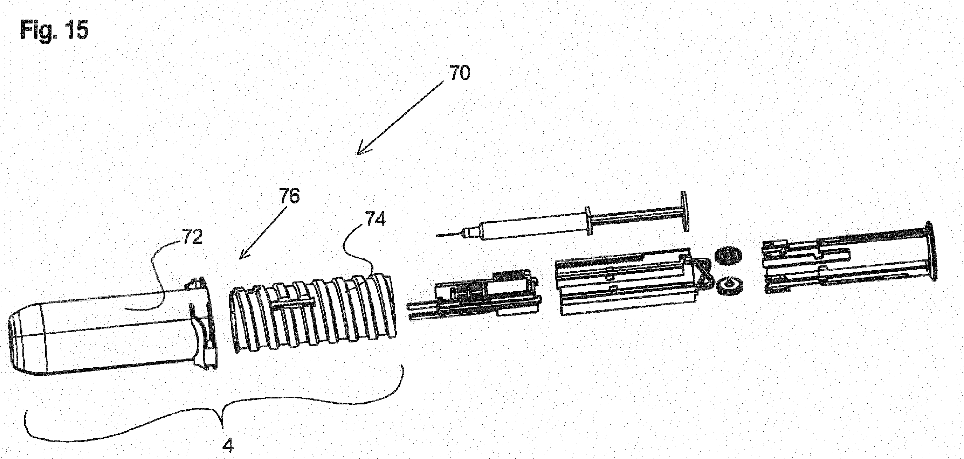

Aus Fig. 15 ist ferner eine für die Injektionsvorrichtung 2; 102 verwendbare Einstechtiefeneinstellung 70 zu entnehmen, die im Wesentlichen aus einem zweiteiligen Gehäuse 4 besteht. Dieses weist einen stirnseitigen ersten Gehäuseteil 72 auf, der zur Einstellung einer gewünschten Einstechtiefe gegenüber einem zweiten Gehäuseteil 74 verlagerbar ist. Hierzu ist zwischen den beiden Gehäuseteilen 72, 74 beispielsweise, wie dargestellt, eine verstellbare Schraubverbindung 76 vorgesehen.

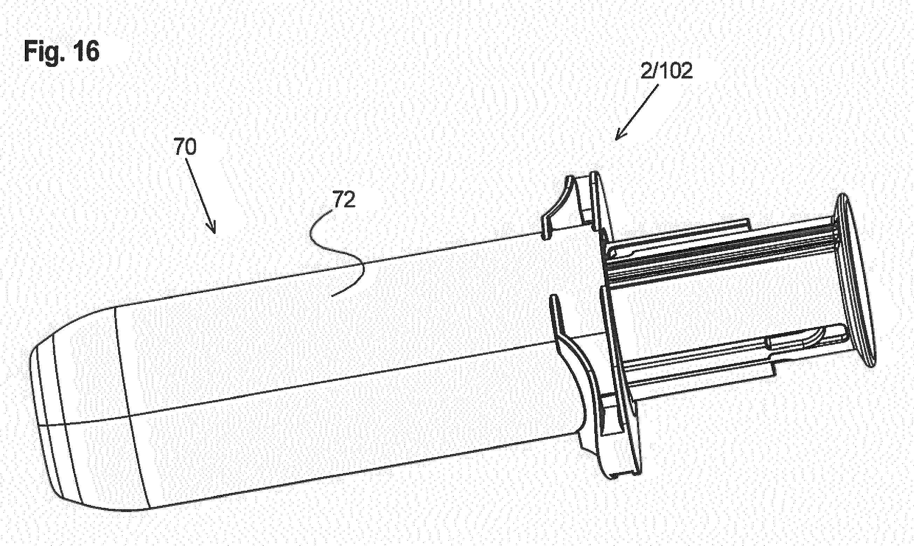

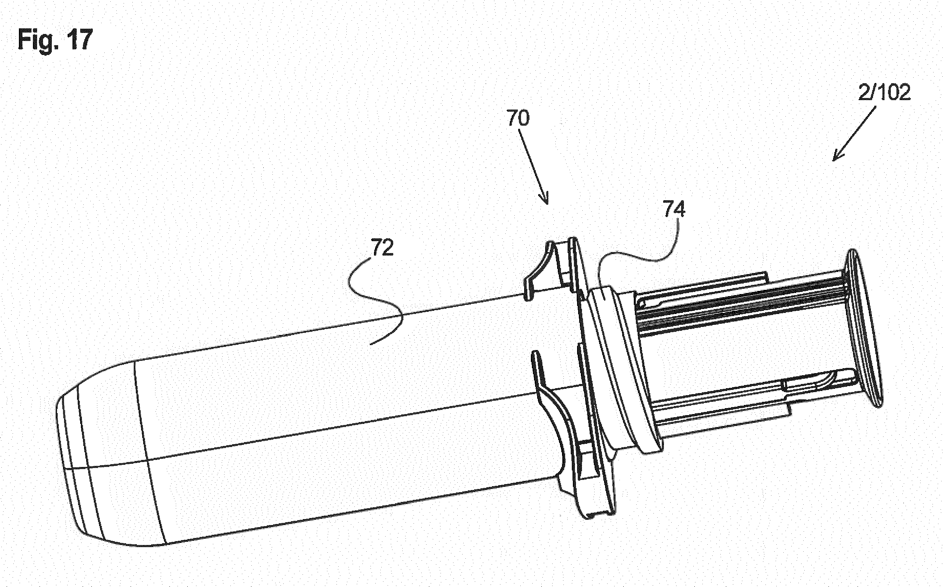

Mittels der Schraubverbindungen 76 kann die Injektionsvorrichtung 2; 102 dabei gegenüber einer Einstellung für eine maximale Einstechtiefe gemäß Fig. 16 in eine Einstellung gemäß Fig. 17 verbracht werden, in der der stirnseitige erste Gehäuseteil 72, der beim Injektionsvorgang als Anschlag an dem betreffenden Körperteil des Patienten dient, weiter nach vorne verbracht ist und die Injektionsnadel 8 dadurch während des Einstechhubes nicht so weit aus dem Gehäuse 4 heraus verlagert werden kann.

Alternativ zu der dargestellten Ausführungsform der Einstechtiefeneinstellung 70 ist dabei auch jede andere Ausführungsform verwendbar, mittels der der erste Gehäuseteil 72 gegenüber dem zweiten Gehäuseteil 74 in verschiedenen Stellungen positioniert werden kann. Beispielsweise kann der erste Gehäuseteil 72 gegenüber dem zweiten Gehäuseteil lateral oder entlang einer Steuerkurve verschiebbar sein oder mittels eines verlagerbaren Betätigungselementes in verschiedene vorbestimmte Positionen verstellbar sein (nicht dargestellt). In einer weiteren alternativen Ausführungsform der Einstechtiefeneinstellung 70 kann auch ein abnehmbarer Abstandshalter verwendet werden, der durch einen anderen Abstandshalter mit unterschiedlichen Längenabmessungen ausgetauscht werden kann, um eine bestimmte Einstechtiefe festzulegen (nicht dargestellt).

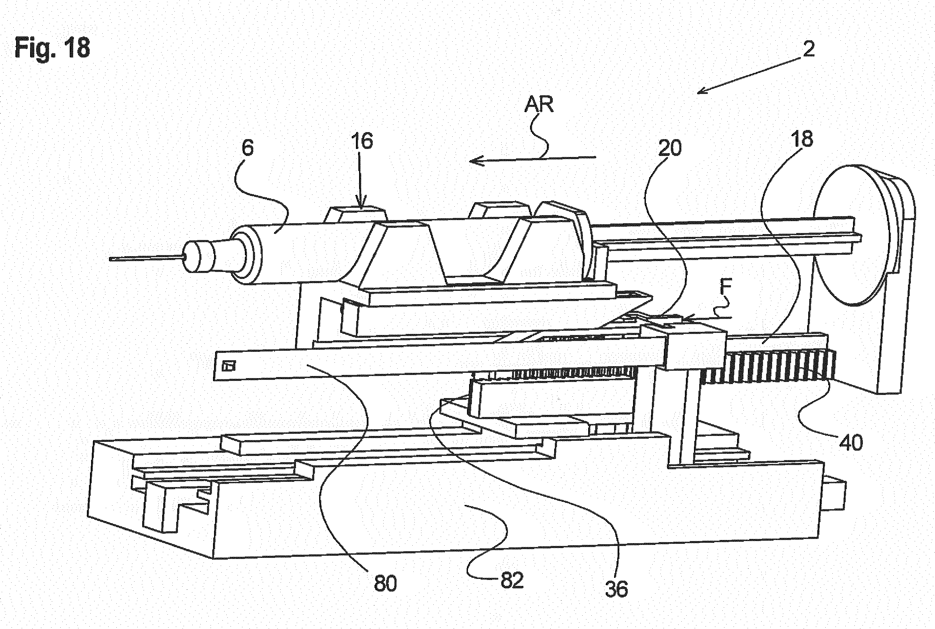

Fig. 18 zeigt eine automatisch betreibbare Ausführungsform der Injektionsvorrichtung 2, bei der die Beaufschlagung des Betätigungselementes 20 mit der Antriebskraft F mittels eines mechanischen Kraftspeichers 80 erfolgt. Das Betätigungselement 20 ist dabei beispielhaft durch eine Zahnradaufnahme gebildet, an der eine Rollfeder als mechanischer Kraftspeicher 80 angreift, die in nicht näher dargestellter Weise an einem Träger 82 der Injektionsvorrichtung 2 gehalten ist.

Zudem ist die Spritzenaufnahme 16 hierbei derart ausgeführt, dass die Spritze 6 nach einer Anwendung durch eine neue Spritze 6 ausgetauscht werden kann, um die Injektionsvorrichtung 2 mehrfach wiederverwenden zu können.

## Patentansprüche

1. Injektionsvorrichtung (2; 102)
mit einem Gehäuse (4), in dem eine Spritze (6) aufnehmbar ist, die eine Injektionsnadel (8), eine Aufnahmekammer (10), einen Kolben (12) und einen Kolbenstößel (14) aufweist,
und einer Steuerungsanordnung (24) zur gesteuerten Betätigung der Spritze (6) während eines Anwendungsablaufes, der wenigstens einen Einstechhub, einen Injektionshub und einen Rückholhub umfasst,
wobei die Steuerungsanordnung (24) ein Betätigungselement (20) zur Beaufschlagung durch eine Antriebskraft (F),
eine gegenüber dem Gehäuse (4) verschiebbare Spritzenaufnahme (16), an der die Aufnahmekammer (10) festlegbar ist,
eine gegenüber dem Gehäuse (4) verschiebbare Stößelaufnahme (18), an der der Kolbenstößel (14) festlegbar ist,
sowie eine Übertragungseinrichtung (26) aufweist, mittels der die Stößelaufnahme (18) mit dem Betätigungselement (20) koppelbar ist, wobei die Übertragungseinrichtung (26) ein Zahnradgetriebe (28) aufweist, mittels dem in Abhängigkeit von einer Relativbewegung des Betätigungselementes (20) gegenüber dem Gehäuse (4) die Stößelaufnahme (18) antreibbar ist,
**dadurch gekennzeichnet, dass** das Zahnradgetriebe (28) hierzu am Gehäuse (4) selbst eine Verzahnung aufweist.

2. Injektionsvorrichtung nach Anspruch 1, **dadurch gekennzeichnet, dass** das Zahnradgetriebe (28) eine erste betätigungselementseitige Verzahnung (32) aufweist, die in Antriebsrichtung (AR) des Betätigungselementes (20) mit einer ersten gehäuseseitigen Verzahnung (36) unter Erzeugung einer ersten Drehrichtung und beabstandet dazu mit einer auf einer anderen Seite des Gehäuses (4) angeordneten zweiten gehäuseseitigen Verzahnung (38) unter Erzeugung einer zweiten Drehrichtung in kämmenden Eingriff bringbar ist, wobei die zweite Drehrichtung entgegengesetzt zur ersten Drehrichtung gerichtet ist.

3. Injektionsvorrichtung nach Anspruch 1 oder 2, **dadurch gekennzeichnet, dass** das Zahnradgetriebe (28) ein am Betätigungselement (20) verdrehbar gelagertes Zahnrad (30) aufweist, das sowohl mit wenigstens einer der gehäuseseitigen Verzahnungen (36; 38) als auch mit einer stößelaufnahmeseitigen Verzahnung (40) in kämmenden Eingriff bringbar ist.

4. Injektionsvorrichtung nach Anspruch 3, **dadurch gekennzeichnet, dass** das Zahnrad (30) zusätzlich mit einer spritzenaufnahmeseitigen Verzahnung (42) in kämmenden Eingriff bringbar ist.

5. Injektionsvorrichtung nach Anspruch 3 oder 4 , **dadurch gekennzeichnet, dass** an dem Zahnrad (30) neben der ersten betätigungselementseitigen Verzahnung (32) wenigstens eine zweite betätigungselementseitige Verzahnung (34) vorgesehen ist und die erste betätigungselementseitige Verzahnung (32) eine erste Zähnezahl und die zweite betätigungselementseitige Verzahnung (34) eine zweite Zähnezahl aufweist, die unterschiedlich zur ersten Zähnezahl ist und die beiden betätigungselementseitigen Verzahnungen (32, 34) in Antriebsrichtung (AR) des Betätigungselementes (20) mit zwei Gegenverzahnungen unter Erzeugung einer Drehbewegung mit unterschiedlichen Übersetzungen in kämmenden Eingriff bringbar sind.

6. Injektionsvorrichtung nach einem der Ansprüche 2 bis 5, **dadurch gekennzeichnet, dass** die betätigungselementeseitigen Verzahnungen (32, 34) und Gegenverzahnungen wenigstens teilweise als Spitzverzahnungen sowie wenigstens teilweise federnd ausgebildet sind und die gehäuseseitigen Verzahnungen (36, 38) bezüglich der Antriebsrichtung (AR) wenigstens bereichsweise ansteigende Zahnhöhen aufweisen.

7. Injektionsvorrichtung nach einem der Ansprüche 1 bis 6, **dadurch gekennzeichnet, dass** an der Stößelaufnahme (18) Ferderelemente (19) vorgesehen sind, mittels denen am Kolbenstößel (14) eine Vorspannung in Antriebsrichtung (AR) anlegbar ist.

8. Injektionsvorrichtung nach einem der Ansprüche 1 bis 7, **dadurch gekennzeichnet, dass** wenigstens ein mit dem Betätigungselement (20) zusammenwirkendes Dämpfungselement vorgesehen ist.

9. Injektionsvorrichtung nach einem der Ansprüche 1 bis 8, **dadurch gekennzeichnet, dass** ein Blockierungsmechanismus (50) vorgesehen ist, mittels dem das Betätigungselement (20) in einer Ausgangsstellung bis zum Erreichen eines Schwellenwertes einer in Antriebsrichtung (AR) wirkenden Antriebskraft (F) blockiert wird.

10. Injektionsvorrichtung nach Anspruch 9, **dadurch gekennzeichnet, dass** der Blockierungsmechanismus (50) eine an einen Anschlag (48) anlegbare Federnase (46) und ein gegen eine Federkraft verschwenkbares Verriegelungselement aufweist.

11. Injektionsvorrichtung nach einem der Ansprüche 1 bis 10, **dadurch gekennzeichnet, dass** die Spritzenaufnahme (16) zur Durchführung des Einstechhubes fest mit der Stößelaufnahme (18) koppelbar ist.

12. Injektionsvorrichtung nach Anspruch 11, **dadurch gekennzeichnet, dass** die Kopplung mittels eines in eine Aufnahme (64) lösbar eingreifenden Eingriffselementes (62) oder eines verlagerbaren Kulissensteins herstellbar ist.

13. Injektionsvorrichtung nach einem der Ansprüche 1 bis 12, **dadurch gekennzeichnet, dass** am Zahnradgetriebe (28) ein Leerhub zur Erzeugung einer Verweilzeit vorgesehen ist, über den hinweg trotz Relativbewegung des Betätigungselementes (20) gegenüber dem Gehäuse (4) keine Antriebskraft (F) auf die Stößelaufnahme (18) übertragbar ist

14. Injektionsvorrichtung nach Anspruch 5, **dadurch gekennzeichnet, dass** die zweite betätigungselementseitige Verzahnung (34) des Zahnrades (30) zur Durchführung des Rückholhubes mit der zweiten Gegenverzahnung in kämmenden Eingriff bringbar ist, die durch die zweite gehäuseseitige Verzahnung (38) gebildet ist,
und die erste betätigungselementeseitige Verzahnung (32) des Zahnrades (30) gleichzeitig mit der stößelaufnahmeseitigen Verzahnung (40) in kämmenden Eingriff bringbar ist,
wobei zudem die Spritzenaufnahme (16) fest mit der Stößelaufnahme (18) koppelbar ist und die Kopplung mittels eines in eine Aufnahme lösbar eingreifenden Federelementes herstellbar ist.

15. Injektionsvorrichtung nach einem der Ansprüche 1 bis 14, **dadurch gekennzeichnet, dass** das Betätigungselement (20) in einer Endstellung, in der der Rückholhub abgeschlossen und die Nadel (8) wieder vollständig innerhalb des Gehäuses (4) angeordnet ist, verriegelbar ist.

16. Injektionsvorrichtung nach einem der Ansprüche 1 bis 15, **dadurch gekennzeichnet, dass** das Gehäuse (4) einen stirnseitigen Gehäuseteil (72) aufweist, der zur Einstellung einer Einstechtiefe gegenüber dem übrigen Gehäuse (4) verlagerbar ist.

17. Injektionsvorrichtung nach einem der Ansprüche 1 bis 16, **dadurch gekennzeichnet, dass** das Betätigungselement (20) über einen mechanischen Kraftspeicher (80) mit der Antriebskraft (F) beaufschlagbar ist.

## Claims

1. Injection device (2; 102)
with a housing (4) in which a syringe (6) can be received that has an injection needle (8), a receiving chamber (10), a piston (12), and a piston ram (14),
and a control arrangement (24) for the controlled actuation of the syringe (6) during an application procedure that includes at least one puncture stroke, one injection stroke, and one return stroke,
wherein the control arrangement (24) has an actuation element (20) for applying a drive force (F)
to a syringe holder (16) which is movable relative to the housing (4) and on which the receiving chamber (10) can be secured,
a ram holder (18) which is movable relative to the housing (4) and on which the piston ram (14) can be secured,
and a transmission mechanism (26), by means of which the ram holder (18) can be coupled to the actuation element (20), wherein the transmission mechanism (26) has a toothed gearing (28), by means of which the ram holder (18) can be driven, according to a relative movement of the actuation element (20) with respect to the housing (4),
**characterised in that** for this purpose the toothed gearing (28) has teeth at the housing (4) itself.

2. Injection device according to claim 1, **characterised in that** the toothed gearing (28) comprises a first teeth arrangement (32) on the actuation element side, which, in the drive direction (AR) of the actuation element (20), can be brought into meshing engagement with a teeth arrangement (36) on the housing side, thereby producing a first direction of rotation, and, at a distance interval from this, can be brought into meshing engagement with a second tooth arrangement (38) on another side of the housing (4), thereby producing a second direction of rotation, wherein the second direction of rotation is directed opposed to the first direction of rotation.

3. Injection device according to claim 1 or 2, **characterised in that** the toothed gearing (28) comprises a toothed wheel (30) rotatably mounted at the actuation element (20), which can be brought into meshing contact both with at least one of the housing side tooth arrangements (36; 38) as well as with a tooth arrangement (40) on the ram holder side.

4. Injection device according to claim 3, **characterised in that** the toothed wheel (30) can additionally be brought into meshing engagement with a tooth arrangement (42) on the syringe holder side.

5. Injection device according to claim 3 or 4, **characterised in that,** in addition to the first actuation element side tooth arrangement (32), at least one second actuation element side tooth arrangement (34) is provided at the toothed wheel (30), and the first actuation element side tooth arrangement (32) has a first number of teeth, and the second actuation element side tooth arrangement (34) has a second number of teeth which is different from the first number of teeth, and the two actuation element side tooth arrangements (32, 34), in the drive direction (AR) of the actuation element (20) can be brought into meshing engagement with two counter-tooth arrangements, producing a rotational movement with different gear transmission ratios.

6. Injection device according to any one of claims 2 to 5, **characterised in that** the actuation element side tooth arrangements (32, 34) and counter-tooth arrangements are configured at least partially as serrated teeth as well as at least partially as spring-loaded, and the housing side tooth arrangements (36, 38) exhibit tooth heights which at least in sections rise in relation to the drive direction (AR).

7. Injection device according to any one of claims 1 to 6, **characterised in that** spring elements (19) are provided at the ram holder (18), by means of which a pre-tension can be imposed at the piston ram (14) in the drive direction (AR).

8. Injection device according to any one of claims 1 to 7, **characterised in that** at least one damping element is provided, interacting with the actuation element (20).

9. Injection device according to any one of claims 1 to 8, **characterised in that** a blocking mechanism (50) is provided, by means of which the actuation element (20) in a starting position is blocked until the attainment of a threshold value of a drive force (F) taking effect in the drive direction (AR).

10. Injection device according to claim 9, **characterised in that** the blocking mechanism (50) comprises a spring nose element (46) and a locking element (48) which can be pivoted against a spring force.

11. Injection device according to any one of claims 1 to 10, **characterised in that** the syringe holder (16) can be securely coupled to the ram holder (18) in order to carry out the puncture stroke.

12. Injection device according to claim 11, **characterised in that** the coupling can be produced by means of an engagement element (62) which engages in a detachable manner into a holder (64), or by means of a displaceable sliding block.

13. Injection device according to any one of claims 1 to 12, **characterised in that** an empty stroke is provided at the toothed gearing (28), in order to produce a dwell time, by means of which, despite the relative movement of the actuation element (20) in relation to the housing (4), no drive force (F) can be transferred onto the ram holder (18).

14. Injection device according to claim 5, **characterised in that** the second actuation element side tooth arrangement (34) of the toothed wheel (30), in order to carry out the return stroke, can be brought into meshing engagement with the second counter-tooth arrangement, which is formed by the second housing side tooth arrangement (38),
and the first actuation element side tooth arrangement (32) of the toothed wheel (30) can be simultaneously brought into meshing engagement with the ram holder side tooth arrangement (40),
wherein, in addition, the syringe holder (16) can be securely coupled to the ram holder (18), and the coupling can be produced by means of a spring element which engages in a detachable manner into a holder.

15. Injection device according to any one of claims 1 to 14, **characterised in that** the actuation element (20) can be locked in an end position, in which the return stroke has been completed and the needle (8) is again arranged entirely inside the housing (4).

16. Injection device according to any one of claims 1 to 15, **characterised in that** the housing (4) comprises a face side housing part (72), which, in order to adjust a puncture depth, can be displaced in relation to the remainder of the housing (4).

17. Injection device according to any one of claims 1 to 16, **characterised in that** the actuation element (20) can be subjected to the drive force (F) by means of a mechanical energy storage mechanism (80).

## Revendications

1. Dispositif d'injection (2 ; 102)
avec un boîtier (4), dans lequel une seringue (6) peut être logée, laquelle comporte une canule d'injection (8), une chambre de réception (10), un piston (12) et un poussoir de piston (14),
et un dispositif de commande (24) pour l'actionnement commandé de la seringue (6) pendant un déroulement d'application comprenant au moins une course de piqûre, une course d'injection et une course de retrait,
le dispositif de commande (24) comprenant un élément d'actionnement (20) pour la sollicitation avec une force d'entrainement (F),
un réceptacle de seringue (16) pouvant être translaté par rapport au boitier (4) et auquel peut être fixé la chambre de réception (10),
un réceptacle de poussoir (18) pouvant être translaté par rapport au boitier (4) et auquel peut être fixé le poussoir de piston (14),
ainsi qu'un moyen de transmission (26) au moyen duquel le réceptacle de poussoir (18) peut être couplé avec l'élément d'actionnement (20), le moyen de transmission (26) comprenant un engrenage (28) au moyen duquel le réceptacle de poussoir (18) peut être entrainé en fonction d'un mouvement relatif de l'élément d'actionnement (20) par rapport au boitier (4),
**caractérisé en ce que** pour cela, l'engrenage (28) présente une denture sur le boitier (4) même.

2. Dispositif d'injection selon la revendication 1, **caractérisé en ce que** l'engrenage (28) présente une première denture (32) côté élément d'actionnement, qui peut dans la direction d'entraiment (AR) de l'élément d'actionnement (20) être mise en engrènement avec une première denture (36) côté boitier en générant une première direction de rotation et, à distance de ceci, avec une deuxième denture (38) côté boitier, agencée sur un autre côté du boitier (4), en générant une deuxième direction de rotation opposée à la première direction de rotation.

3. Dispositif d'injection selon la revendication 1 ou 2, **caractérisé en ce que** l'engrenage (28) présente un pignon (30) monté rotatif sur l'élément d'actionnement (20), le pignon (30) pouvant être mis en engrènement avec l'une au moins des dentures (36; 38) côté boitier et avec une denture (40) côté réceptacle du poussoir.

4. Dispositif d'injection selon la revendication 3, **caractérisé en ce que** le pignon (30) peut en outre être mis en engrènement avec une denture (42) côté réceptacle de seringue.

5. Dispositif selon la revendication 3 ou 4, **caractérisé en ce que** sur le pignon (30) est prévue, en plus de la première denture (32) côté élément d'actionnement, au moins une deuxième denture (34) côté élément d'actionnement, et la première denture (32) côté élément d'actionnement présente un premier nombre de dents et la deuxième denture (34) côté élément d'actionnement présente un deuxième nombre de dents différent du premier nombre de dents et les deux dentures (32 ; 34) côté élément d'actionnement peuvent être mises en engrènement dans la direction d'entrainement (AR) de l'élément d'actionnement (20) avec deux dentures conjuguées en générant un mouvement de rotation avec des rapports de transmission différents.

6. Dispositif d'injection selon l'une des revendications 2 à 5, **caractérisé en ce que** les dentures (32, 34) côté élément d'actionnement et les dentures conjuguées sont formées au moins partiellement comme dentures striées et au moins partiellement élastiques et les dentures (36, 38) côté boitier présentent au moins par endroits des hauteurs de dents croissantes par rapport à la direction d'entrainement (AR).

7. Dispositif d'injection selon l'une des revendications 1 à 6, **caractérisé en ce que** des éléments ressort (19) sont prévus sur le réceptacle de poussoir (18), au moyen desquels une pré-contrainte peut être appliquée au poussoir de piston (14) dans la direction d'entrainement (AR).

8. Dispositif d'injection selon l'une des revendications 1 à 7, **caractérisé en ce qu'**il est prévu au moins un élément d'amortissement coopérant avec l'élément d'entrainement (20).

9. Dispositif d'injection selon l'une des revendications 1 à 8, **caractérisé en ce qu'**il est prévu un mécanisme de blocage (50) au moyen duquel l'élément d'actionnement (20) est bloqué dans une position initiale jusqu'à ce qu'une force d'entrainement (F) agissant dans la direction d'entrainement (AR) atteigne une valeur de seuil.

10. Dispositif d'injection selon la revendication 9, **caractérisé en ce que** le mécanisme de blocage (50) comporte une languette ressort (46) pouvant être appuyée contre une butée (48) et un élément de verrouillage pouvant être pivoté contre une force élastique.

11. Dispositif d'injection selon l'une des revendications 1 à 10, **caractérisé en ce que** le réceptacle de seringue (16) peut être rigidement couplé avec le réceptacle de poussoir (18) pour la réalisation de la course de piqûre.

12. Dispositif d'injection selon la revendication 11, **caractérisé en ce que** le couplage peut être établi au moyen d'un élément de prise (62) venant en prise de manière amovible dans un logement, ou au moyen d'un coulisseau déplaçable.

13. Dispositif d'injection selon l'une des revendications 1 à 12, **caractérisé en ce que** l'engrenage (28) est prévu avec une course à vide pour générer un temps d'arrêt pendant lequel aucune force d'entrainement (F) ne peut être transmise au réceptacle de poussoir (18) malgré un mouvement relatif de l'élément d'actionnement (20) par rapport au boitier (4).

14. Dispositif d'injection selon la revendication 5, **caractérisé en ce que** la deuxième denture (34) côté élément d'actionnement, du pignon (30), peut être mise en engrènement, pour la réalisation de la course de retrait, avec la deuxième denture conjuguée formée par la deuxième denture (38) côté boitier,
et la première denture (32) côté élément d'actionnement, du pignon (30), peut en même temps être mise en engrènement avec la denture (40) côté réceptacle du poussoir,
le réceptacle de seringue (16) pouvant en outre être couplé rigidement avec le réceptacle du poussoir (18) et le couplage peut être établi au moyen d'un élément à ressort s'engageant de manière amovible dans un logement.

15. Dispositif d'injection selon l'une des revendications 1 à 14, **caractérisé en ce que** l'élément d'actionnement (20) peut être verrouillé dans une position terminale, dans laquelle la course de retrait est terminée et la canule (8) est de nouveau complètement logée dans le boitier (4).

16. Dispositif d'injection selon l'une des revendications 1 à 15, **caractérisé en ce que** le boitier (4) comporte une partie frontale (72) pouvant être déplacée pour le réglage d'une profondeur de piqûre par rapport au reste du boitier (4).

17. Dispositif d'injection selon l'une des revendications 1 à 16, **caractérisé en ce que** l'élément d'actionnement (20) peut être soumis à la force d'entrainement (F) par un accumulateur de force (80) mécanique.
